Europäisches Patentamt

European Patent Office

Office européen des brevets

(11) Numéro de publication : **0 445 043 A1**

(12) # DEMANDE DE BREVET EUROPEEN

(21) Numéro de dépôt : 91400579.8

(22) Date de dépôt : 01.03.91

(51) Int. Cl.⁵ : **C02F 3/28, C02F 3/10**

(30) Priorité : 02.03.90 FR 9002689

(43) Date de publication de la demande :
04.09.91 Bulletin 91/36

(84) Etats contractants désignés :
AT BE CH DE DK ES FR GB GR IT LI LU NL SE

(71) Demandeur : CENTRE NATIONAL DE LA
RECHERCHE SCIENTIFIQUE (CNRS)
15, Quai Anatole France
F-75007 Paris (FR)

(72) Inventeur : **Junter, Guy-Alain**
Le Bosc-Nouvel Valmartin-Le Boasse
F-76690 Clekes (FR)
Inventeur : **Lemoine, Denis**
Résidence Effel, 2, rue Jean Effel
F-76150 Maromme (FR)
Inventeur : **Jouenne, Thierry**
17B Avenue Jacques Chastellain
F-76000 Rouen (FR)

(74) Mandataire : **Phélip, Bruno et al**
c/o Cabinet Harlé & Phélip 21, rue de La
Rochefoucauld
F-75009 Paris (FR)

(54) **Procédé biologique pour la dénitrification des milieux liquides et appareillage pour la mise en oeuvre de ce procédé.**

(57)   Procédé biologique pour la dénitrification de milieux liquides et notamment d'eau destinée à la consommation humaine par réduction des nitrates à l'aide d'une source de carbone et d'un système biologique immobilisé . Les nitrates et la source de carbone sont fournis au système biologique immobilisé, qui peut être tout microorganisme capable de respirer les nitrates en anaérobiose notamment une bactérie du type Pseudomonas, sous la forme de deux flux séparés.

L'immobilisation des bactéries est effectuée préférentiellement dans un gel du type agar dont l'épaisseur tient compte de la nature et des concentrations des substrats et du gel circonscrit par deux membranes microporeuses.

La source de carbone peut être du méthanol ou des effluents industriels ou eaux usées contenant des composés organiques .

EP 0 445 043 A1

FIG.1

EP 0 445 043 A1

## PROCEDE BIOLOGIQUE POUR LA DENITRIFICATION DES MILIEUX LIQUIDES ET APPAREILLAGE POUR LA MISE EN OEUVRE DE CE PROCEDE

La présente invention concerne un procédé biologique pour la dénitrification de milieux liquides. Elle a également pour objet un appareillage pour la mise en oeuvre d'un tel procédé.

L'accumulation de nitrates dans l'eau est un problème grave dans de nombreux pays et plus particulièrement dans les pays à agriculture intensive. Parmi les différentes techniques disponibles pour l'élimination des nitrates de l'eau, seules les techniques de dénitrification par échange d'ions et la dénitrification bioloqigue sont considérées comme étant possibles à mettre en oeuvre à grande échelle.

De nombreuses difficultés pratiques sont rencontrées dans le cas des procédés par échange d'ions, notamment l'encrassement des résines, ainsi que la production de grandes quantités de déchets,les nitrates n'étant pas éliminés.

La dénitrification biologique apparaît donc comme la technique la plus adaptée à l'élimination des nitrates. Dans la plupart des cas, une source carbonée organique doit être ajoutée à l'effluent. L'éthanol et l'acide acétique sont utilisés communément à cet usage.Cependant, en raison des contaminations microbiologiques et chimiques, cette technique de dénitrification conduit à des effluents de mauvaise qualité, nécessitant ainsi des traitements ultérieurs afin de garantir la qualité de l'eau, et en particulier de l'eau destinée à la consommation humaine.

L'immobilisation des microorganismes effectuant la dénitrification permet de supprimer en partie les effets néfastes de ce type de traitement, en réduisant la fuite des microorganismes dans le milieu traité. Différentes études sur la dénitrification de l'eau par des microorganismes immobilisés ont déjà été publiées (Nilsson et al. European J. Appl. Microbiol, 1980, 10: 261-274; Vossoughi et al., Water Res., 1982, 16: 995-1002; Lemoine et al, Biotechnol. Lett., 1988, 10 399-402, Kokufuta et al. J. Ferment. Technol. 1986 ,64,533-538).

Un procédé original a été développé dans lequel les microorganismes sont piégés dans une couche d'agar associée à une membrane microporeuse. Dans ce système qui est une application de l'invention ayant fait l'objet du brevet français 86 05 633, la membrane joue le rôle de filtre évitant ainsi la fuite des microorganismes (bactéries de l'espèce Pseudomonas putrefaciens), du gel de polysaccharide durant les périodes d'incubation (Lemoine et al. Biotechnol. Lett., 1988, 10, 399-402). De l'eau contenant un fort taux de nitrate (3 mM) complétée avec du méthanol comme source de carbone est traitée et on observe une disparition des nitrates sans production d'ions ammonium. Les taux de dénitrification dans cette étude sont, lors de l'utilisation de méthanol comme source de carbone, de 90 micro moles d'ions nitrate éliminés par jour et par gramme de gel d'agar.

Le système de dénitrification décrit dans cette publication ne résoud cependant pas le problème de la contamination de l'eau à épurer par la source de carbone.

Des réacteurs à double flux sont déjà connus dans l'art antérieur dans des utilisations qui sont différentes de la dénitrification de l'eau et qui ne permettent pas l'immobilisation de microorganismes.

La demande de brevet GB 2 164 663 décrit un réacteur à double flux dans lequel des enzymes ou des bactéries sont retenues dans l'espace formé par deux membranes microporeuses, ces membranes étant au contact des deux flux contenant les réactifs. Outre le fait qu'aucune application ne soit réellement décrite concernant des microorganismes, il n'est pas fait mention d'immobilisation d'enzymes ou de microorganismes. En effet, selon une mise en oeuvre préférentielle de cette invention, les enzymes sont simplement adsorbées sur une surface appelée "spacer" située dans l'intervalle compris entre les deux membranes. Cet appareillage, avec ou sans "spacer ", ne permet donc en aucune façon une immobilisation effective des agents biologiquement actifs.

De même, dans la demande EP-A-0.000.461 qui a pour objet un procédé de dénitrification d'effluents, les bactéries dénitrifiantes utilisées sont simplement adsorbées sur un support neutre insoluble constitué par exemple de billes de calcaire ou de billes de charbon enrobées dans du calcaire.

L'immobilisation des microorganismes présente de nombreux avantages qui ont été largement décrits dans l'art antérieur et notamment dans la demande de brevet française 86 05 633. En particulier, lorsque des microorganismes sont immobilisés dans un support permettant leur croissance,la population cellulaire interne augmente quand les microorganismes sont mis en présence d'un substrat. On peut ainsi parvenir à un haut niveau d'activité enzymatique cellulaire. En outre, il est bien établi que l'immobilisation assure une stabilisation dans le temps de l'activité cellulaire.

L'objet de la présente invention est de permettre la dénitrification de liquides et notamment de l'eau destinée à la consommation humaine,en évitant la pollution de ces liquides par les microorganismes et par la source carbonée indispensable à une dénitrification par ces microorganismes tout en procurant aux microorganismes des conditions physiologiques adéquates.

Selon l'invention, on a constaté qu'une telle dénitrification pouvait être mise en oeuvre à l'aide d'un système

3

EP 0 445 043 A1

à double flux permettant ainsi la séparation des flux de liquide à dénitrifier et de liquide contenant la source carbonée. Il est d'autre part apparu de manière surprenante que, outre les effets positifs sur les microorganismes précédemment cités, l'immobilisation des microorganismes dans ce système à double flux permettait un contrôle de leur activité et donc une optimisation de la dénitrification microbiologique et que l'activité de ce système dépendait de facteurs liés à la diffusion des effecteurs du système biologique, et en particulier des substrats, dans le support.

La présente invention a donc pour objet un procédé biologique pour la dénitrification de milieux liquides par réduction des nitrates à l'aide d'une source de carbone et d'un système biologique immobilisé, capable d'assurer la dénitrification, avec lequel on met en contact le liquide à dénitrifier, caractérisé en ce que les nitrates et la source de carbone sont fournis sous la forme de deux flux liquides audit système biologique et en ce qu'on fixe à une valeur comprise entre deux seuils prédéterminés, l'épaisseur de la couche d'immobilisation du système biologique.

Pour chaque situation on déterminera par une expérience de routine les seuils limites,qui dépendent de la nature et de la concentration du gel et des substrats.

Le fait de fournir sous la forme de flux séparés les nitrates et la source de carbone évite la contamination des milieux liquides contenant ces nitrates par des composés carbonés nuisant à la qualité des milieux dénitrifiés.

Le système biologique de dénitrification peut être immobilisé par piégeage dans un support situé entre les deux flux liquides qui peut être circonscrit par deux membranes au contact respectif des deux flux liquides et ayant une porosité permettant la libre circulation des substrats et des produits de la réaction de dénitrification tout en retenant le système biologique. Le piégeage du système biologique permet d'éviter sa déssimination dans les deux flux de milieux contenant des nitrates et des sources de carbone et a aussi une influence sur son activité.

Le fait que ce support soit circonscrit par deux membranes , permet de réduire encore plus la fuite du système biologique dans les milieux.

L'immobilisation dans un support homogène du système biologique permet une autorégulation de l'activité de ce système en fonction de l'intensité des deux flux.

Ce système biologique de dénitrification peut notamment être au moins un microorganisme et préférentiellement des bactéries du genre Pseudomonas en particulier Pseudomonas denitrificans. Tout autre type de microorganismes capable de respirer les nitrates en anaérobiose convient, par exemple ceux cités par Knowles (Microbiol. Review , 1982, 46, 43-70).Dans ce cas , les microorganismes développent leur activité maximale dans la zone du support où les deux flux vont se rencontrer et il se forme un gradient de dénitrification dans l'épaisseur de support .

Contrairement aux procédés dans lesquels les microorganismes ne sont pas immobilisés, ou sont adsorbés de manière non homogène, le procédé de la présente invention permet une modulation dans l'espace de l'activité du système biologique .

Il est ainsi possible de contrôler le rapport des deux flux afin de permettre une activité optimale du système de dénitrification en influant sur le débit de l'un ou de l'ensemble des deux flux.

Le support dans lequel est piégé le système biologique peut notamment être un gel de polysaccharides et en particulier un gel d'agar et la membrane peut avoir une taille de pores comprises entre 0,2 et 1 micron environ.

La source de carbone peut être du méthanol, de l'acide acétique ,des sucres réducteurs, des composés aromatiques, ou toute source de carbone organique servant de donneurs d'électrons et notamment des effluents industriels ou eaux usées contenant des composés organiques. Dans ce cas, l'utilisation de ces effluents permettra une épuration à moindre coût des milieux à dénitrifier et l'épuration desdits effluents par baisse de la DBO (Demande Biochimique en Oxygène ).

Les milieux à dénitrifier peuvent notamment être de l'eau destinée à la consommation humaine ou des eaux de régénération des colonnes de dénitrification échangeuses d'ions .

Le système biologique peut aussi être un complexe multi-enzymatique comprenant les enzymes nécessaires à la dénitrification des milieux liquides.

Afin d'assurer une activité optimale du système de dénitrification le rapport C/N de la quantité molaire de source de carbone par rapport à la quantité molaire de nitrates doit être comprise préférentiellement entre 2 et 6 et en particulier entre 2,5 et 3,5. Ce rapport est ajusté par réglage de la quantité de l'un ou l'autre des flux.

L'invention a d'autre part pour objet un appareillage pour la mise en oeuvre du procédé de dénitrification comprenant deux compartiments séparés par une couche de support homogène (1) du système biologiquement actif, circonscrite sur chacune de ses deux faces par une membrane microporeuse (2) et au moins deux orifices d'entrée (6) et de sortie (7) respectifs des flux dans les compartiments.

Préférentiellement, le support est constitué d'une couche d'un gel de polysaccharide et en particulier d'un

4

gel d'agar , d'une épaisseur comprise entre 1 et 10 mm et de préférence entre 2 et 5 mm.

L'épaisseur de la couche de support est importante dans ce type de réacteur à double flux du fait des contraintes de diffusion qui empêchent la pénétration des substrats en profondeur dans le support .

La description qui suit donne à titre illustratif et non limitatif des exemples d'application de l'invention.

La figure 1 est une vue éclatée d'un appareillage de type bioréacteur destiné à la dénitrification de milieux aqueux.

La figure 2 représente une coupe schématique longitudinale de cet appareillage.

Les figures 3 et 4 représentent l'évolution en fonction du temps, de la concentration en ions nitrate et nitrite dans le compartiment des milieux à dénitrifier et en ions acétate dans le compartiment de la source de carbone.

La figure 5 représente l'évolution en fonction du temps des concentrations en ions nitrates et nitrites dans les compartiments contenant le milieu à dénitrier et le surnageant de boue contenant la source de carbone .

## EXEMPLE 1

### Procédé et dispositif de dénitrification

#### a) souche de bactéries et conditions de culture.

Une souche de Pseudomonas denitrificans déposée auprès de la Collection Nationale de l'Institut Pasteur sous le n° 63-54, est cultivée sur le milieu de Mueller-Hinton solide et incubée à 30°C durant 36 heures.

Les colonies obtenues sont repiquées et resuspendues dans 70 ml de milieu de Mueller-Hinton liquide et incubées à 30°C dans un bain marie à agitation . Après incubation les cultures sont centrifugées 10 minutes à 2000g, et le culot obtenu est resuspendu dans de l'eau distillée stérile.

#### b) immobilisation des bactéries.

0,36 g d'agar est dissous dans 19 cm³ d'eau distillée par chauffage de la suspension à 100°C. Après refroidissement à 45°C la solution est mélangée avec 1 cm³ d'une suspension cellulaire dense.

11 cm³ de ce mélange sont versés dans la cavité rectangulaire d'un moule obtenu en superposant deux plaques de plastique rigide, la partie supérieure de ce dispositif ayant une ouverture rectangulaire. Le moule est fermé par une autre plaque rigide de plastique avant que le mélange cellules-agar ne durcisse. La couche d'agar biocatalytique obtenue a les caractéristiques suivantes :

- contenu en agar : 1,8% en poids/ volume
- longueur : 185 mm
- largeur : 20 mm
- épaisseur : de 3 à 7 mm
- surface : 37 cm²
- contenu cellulaire : environ 10⁸ cellules/cm³ de gel correspondant à environ 0,02 mg de cellules en poids sec par cm³ de gel

#### c)prétraitement de la couche d'agar

La couche d'agar est incubée dans le milieu de Mueller - Hinton durant 36 heures à la température ambiante. L'ensemble du dispositif est alors lavé par immersion durant 24 heures dans de l'eau de source.

#### d)description de l'appareillage

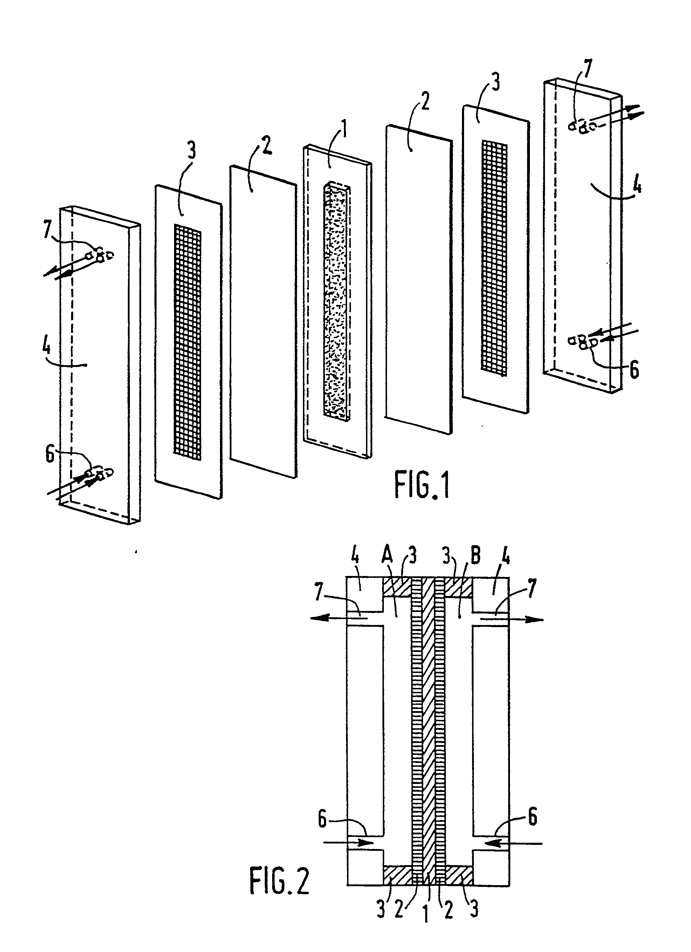

Les figures 1 et 2 représentent un bioréacteur constitué d'une couche de support homogène (1) circonscrite sur chacune de ses deux faces par des membranes microporeuses (2) sur lesquelles sont positionnées des pièces intercalaires (3) assurant la libre circulation des flux liquides apportés par les orifices d'entrée (6) et de sortie (7) des flux aménagés dans les pièces de maintien (4). Ces deux pièces intercalaires forment ainsi deux compartiments A et B correspondant respectivement au compartiment de l'eau à dénitrifier et au compartiment de la solution aqueuse contenant la source de carbone.

Les membranes microporeuses ont une taille de pores de 0,45 microns (Millipore type Durapore).

#### e)conditions expérimentales

Les solutions sont stockées dans des réservoirs externes , dans lesquels un barbotage d'hélium assure des conditions d'incubation anaérobies. Les expérimentations sont mises en oeuvre à la température ambiante (20°C).

L'acide acétique est utilisé comme source de carbone à une concentration comprise entre 2,5 mM et 15 mM selon le rapport carbone/azote (C/N)voulu. Il est dilué dans de l'eau distillée stérile .

Durant les expériences préliminaires effectuées dans le but de déterminer l'épaisseur optimale de la couche d'agar, les solutions contenant l'acétate et les nitrates sont recyclées en utilisant une pompe péristaltique ayant un débit de 40 cm³ par heure .Pour les autres essais , les circuits sont ouverts et les solutions aqueuses circulent de manière continue sans recyclage, le milieu frais étant injecté à la base des compartiments. Dans toutes ces expérimentations le même débit est appliqué dans les deux chambres.

Le milieu à dénitrifier consiste en de l'eau de source en bouteille (pH 7) complémentée avec du $NaNO_3$ à 2,5 mM. Le pH des solutions est ajusté à 6,3.

## f)procédure d'analyse

Les quantités de nitrate et de nitrite dans les milieux liquides sont déterminées à l'aide d'une colonne HPLC à échange d'anions (Hamilton PRPX 100) . La phase mobile est constituée par le sel de sodium de l'acide para hydroxybenzoïque à une concentration de 4 mM, à pH 8,5.

La colonne HPLC est couplée à un détecteur conductrimétrique de type LDC Milton-Roy .

La quantité d'acétate dans les milieux liquides est détectée par le même équipement, avec une phase mobile composée d'hydroxyde de potassium à une concentration de 3,2 mM, à pH 11,5.

## EXEMPLE 2:

### Détermination de l'épaisseur optimale de la couche d'agar .

Le tableau 1 résume les résultats obtenus avec différentes épaisseurs de la couche d'agar,l'expérimentation ayant été effectuée en circuit fermé.

On remarque que l'on obtient la meilleure activité dénitrifiante avec une couche d'agar d'une épaisseur de 3 mm tandis qu'aucune consommation de nitrate n'est détectée avec une couche d'agar de 7 mm.

Les expérimentations décrites dans les exemples suivants ont donc été effectuées avec une épaisseur de 3 mm .

## EXEMPLE 3:

### Cinétique de la réaction dans un réacteur comprenant deux compartiments contenant respectivement une solution de nitrate et une solution d'acétate .

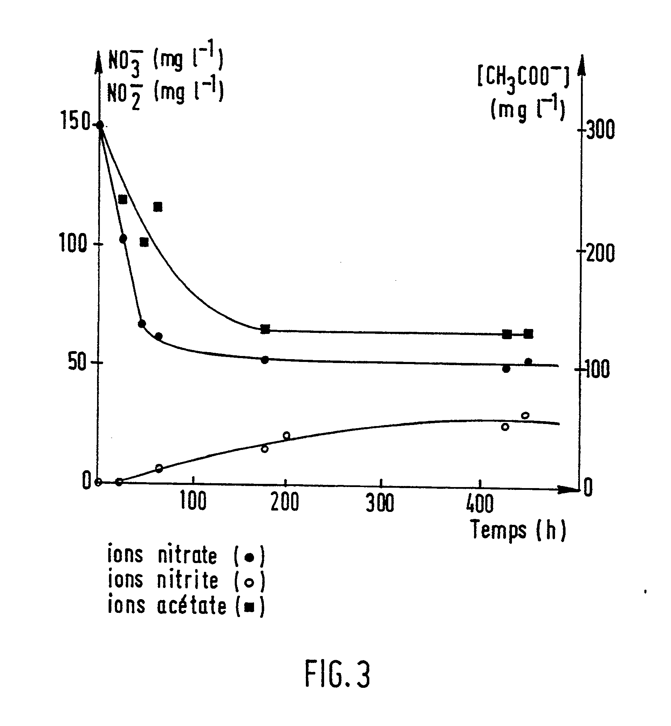

L'évolution de la concentration des ions nitrates et nitrites dans l'eau traitée est montrée sur la figure 3 représentant l'évolution des concentrations en nitrate , en nitrite dans le compartiment A et en acétate dans le compartiment B en fonction du temps exprimé en heures,dans un système en continu.

On remarque sur cette figure que la concentration en nitrate décroît durant la première semaine de l'expérience et se stabilise ensuite . Cette activité dénitrifiante est stable durant 18 jours sans contamination microbienne notable de l'eau traitée.

L'évolution en fonction du temps de la concentration en acétate dans le compartiment B est à peu près similaire à celle des nitrates dans le compartiment A.

La diminution de la concentration en nitrate dans le compartiment A est accompagnée d'une augmentation de la concentration en ions nitrites ($NO_2$-).

Les évolutions des concentrations en ions nitrite et nitrate dans le compartiment B ont aussi été mesurées , et comparées avec les évolutions de ces espèces dans le compartiment A.

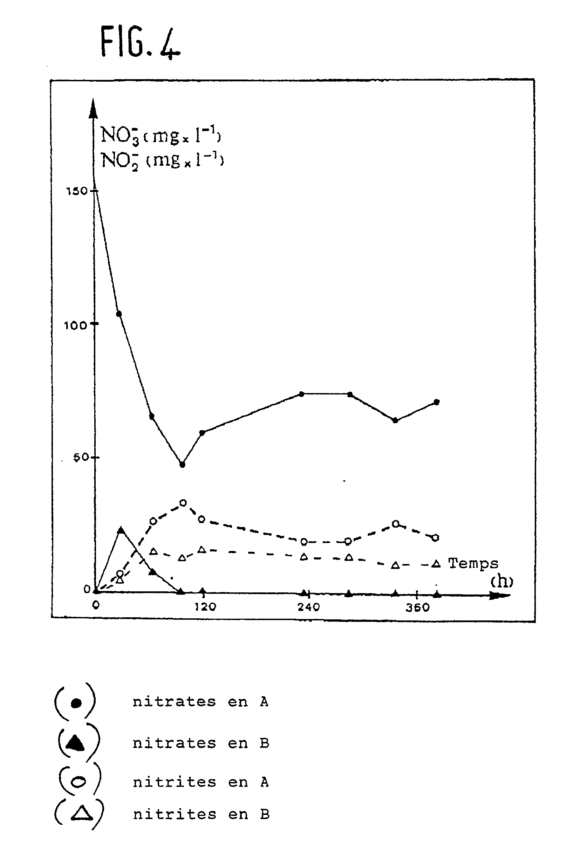

La figure 4 reprend ces résultats . Comme sur la figure 3 , on note une diminution rapide de la concentration en nitrate dans le compartiment A jusqu'à une valeur minimale , puis une stabilisation de ladite concentration . On notera cependant que sur cette figure la concentration semble après la brusque diminution remonter légèrement aux environs de 120 minutes.

De même , cette diminution de la concentration en ions nitrate s'accompagne de l'apparition d'ions nitrites et d'une augmentation du pH , qui se stabilise ensuite.

L'intérêt de cette figure réside dans l'évolution des différents ions dans le compartiment B. On remarque que les ions nitrate ayant diffusé à partir du compartiment A disparaissent rapidement du compartiment B tandis qu'une quantité constante d'ions nitrite est détectée dans le compartiment B .

6

Aucune trace d'acétate n'est observée dans le compartiment A.

## EXEMPLE 4

Influence du rapport C/N sur l'efficacité du réacteur

Afin d'étudier l'effet du rapport C/N sur le procédé d'élimination des nitrates , le réacteur est utilisé en continu avec différentes valeurs de ce paramètre. Les résultats sont présentés dans le tableau 2.

La capacité du réacteur exprimée en micromoles d'ions nitrate ($NO_3$-)complètement réduits par jour et par $cm^2$ de surface active est calculée à partir du niveau d'activité rémanente.

On remarque sur ce tableau qu'un rapport C/N de 3 est optimal en ce qui concerne l'activité du réacteur. Avec un débit pour les deux solutions de 7,5 $cm^3$ par heure , le dispositif réduit complètement 5,2 micromoles d'ions nitrate par jour et par $cm^2$ de surface active avec une efficacité de 43%.

## EXEMPLE 5-

Influence du débit des liquides sur les performances du réacteur.

Ces expérimentations sont effectuées en continu avec une couche d'agar d'une épaisseur de 3 mm et un rapport C/N de 3. Une faible influence du débit sur les performances du réacteur a été remarquée comme l'indique le tableau 3. Dans les conditions optimales, c'est-à-dire avec un débit de 17 $cm^3$/heure , on observe une faible pollution du compartiment A par l'acide acétique, correspondant à 5% de la concentration mesurée dans le compartiment B.

De plus, aucun ion nitrate n'est retrouvé dans le compartiment contenant l'acide acétique (compartiment B ) . La présence des ions nitrites est détectée dans les deux compartiments à raison d'environ 11 mg /$dm^3$.

Les résultats décrits ci-dessus montrent que le procédé de dénitrification selon l'invention permet d'obtenir une élimination des nitrates comparable à celle observée précédemment dans des réactions à simple flux , quoique l'on observe des taux de dénitrification rémanents inférieurs à ceux précédemment observés . Ces différences sont probablement dues aux limitations de diffusion des deux substrats à travers les membranes microporeuses et la couche d'agar.

En ce qui concerne les contaminations chimiques de chacun des compartiments par les substrats de l'autre compartiment , on remarque que la fuite d'acétate dans le compartiment des nitrates (compartiment A) est négligeable. Les ions nitrites sont retrouvés dans les deux compartiments du réacteur.

## EXEMPLE 6 :

Dénitrification en continu par deux réacteurs en série utilisant l'acétate comme source de carbone .

Un réacteur appelé second réacteur a été branché en aval d'un premier réacteur . Les structures unitaires sont identiques à celles décrites dans les exemples précédents .

La source de carbone est l'acétate et est contenue initialement dans les compartiments B des deux réacteurs .

Les concentrations des diverses espèces ioniques en solution ont été mesurées aux entrées et sorties des deux compartiments A et B de chaque réacteur .

Les résultats sont résumés dans le tableau 4 ci-après dans lequel figurent les concentrations en ions nitrate , nitrite et acétate .

On remarque que la plupart des ions nitrites sont éliminés du flux d'acétate par ce système double , tandis que la concentration de ces ions dans les effluents nitratés du second réacteur est égale à celle à l'entrée de ce réacteur . Cependant, une diminution notable du taux d'ions nitrate dans la solution à traiter est observée .

Le processus de dénitrification a été d'autre part caractérisé par les paramètres suivants qui reflètent mieux l'état de fonctionnement en continu ( régime permanent ) :

– le taux de dénitratation c'est-à-dire le taux d'élimination des ions nitrates de la solution aqueuse nitratée;( en µg/h)

– le taux de dénitrification , c'est-à-dire le taux d'élimination des ions nitrates sous forme de gaz ,( en µg/h),

– l'efficacité du procédé de dénitrification définie comme le rapport en pourcentage de la quantité d'ions nitrates réduite sous forme de gaz sur la quantité d'ions nitrates fournie par le flux aqueux nitraté par unité de temps; cette efficacité correspond en fait au rapport du taux de dénitrification sur le taux auquel les ions nitrates sont fournis ,

– le rendement de dénitrification exprimé comme le rapport en pourcentage du taux de dénitrification expérimental sur le taux de dénitrification théorique calculé à partir du taux de consommation expérimentale de l'acétate selon la réaction suivante :

$$5\ CH_3COO^- \longrightarrow 10\ CO_2 + 4\ N_2 + 13\ OH^-$$
$$+\ 8\ NO_3^-\ (+\ 13\ Na^+) \qquad (\ +\ 13\ Na^+\ ) +\ H_2O$$

Les résultats sont représentés dans le tableau 5 ci-après .

Ce tableau 5 montre que l'activité globale des cellules immobilisées est inférieure dans le deuxième réacteur par rapport au premier réacteur.

L'efficacité de la dénitrification est cependant supérieure dans le second réacteur . L'efficacité de l'ensemble du système atteint 64 %.

## EXEMPLE 7 -

### Activité dénitrifiante en présence d'ions phosphate .

Le dispositif utilisé dans cet exemple est un dispositif composé d'un seul réacteur comme décrit précédemment , utilisant comme source de carbone une solution d'acétate .

Les ions phosphates sont ajoutés dans la solution d'acétate sous forme de phosphate de sodium ( 10 ppm) .

Le tableau 6 ci-après illustre cet exemple .

Les taux de dénitrification et de dénitratation ont été définis dans l'exemple précédent .

Ces résultats montrent que le taux de dénitrification est supérieur en présence d'ions phosphates au taux obtenu en absence de ces ions .

Par contre , ces ions exercent des effets opposés sur le taux de dénitratation.

Des faibles concentrations en nitrites ( 5 mg/dm³ ) sont détectées dans les eaux traitées sortant du réacteur , tandis que les effluents contenant la source de carbone en sont libres.

## EXEMPLE 8 :

### Activité dénitrifiante d'un réacteur simple utilisant comme source de carbone des surnageants de boues.

Les surnageants de boues sont des surnageants non chlorés obtenus à partir de réservoir de décantation d'installations de traitement des eaux usées. Avant utilisation les surnageants sont filtrés et dilués deux fois avec de l'eau de source .

Les cinétiques d'évolution des concentrations ioniques en solution ont été mesurées en fonction du temps dans les compartiments A et B de ce réacteur.

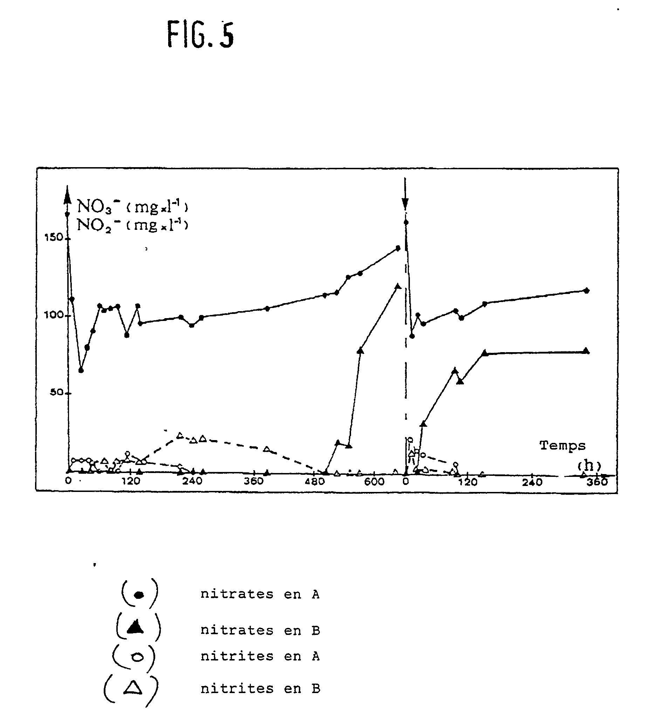

La figure 5 illustre cette expérimentation .Les concentrations des ions nitrates et nitrites dans les compartiments A et B ont été mentionnées . Le flux circulant dans ce réacteur est de 10 cm³/h .

Cette figure montre que la cinétique de dénitrification est similaire à celle observée précédemment en fournissant aux cellules immobilisées de l'acétate .

On observe que le niveau maximum d'activité est rapidement atteint et est suivi par une période de diminution d'activité . Ensuite , l'activité reste stable durant environ 3 semaines avant de chuter brutalement . Une importante quantité d'ions nitrates apparaît alors dans l'effluent de la source de carbone comme conséquence de la baisse d'activité du réacteur . Cependant , aucune pollution du compartiment A par les eaux usées du compartiment B n'est détectée durant les trois semaines d'activité dénitrifiante stable.

En dépit de l'effet négatif dû à la demande biochimique d'oxygène ( DBO ) importante de l'eau, exercée sur la stabilité à long terme de l'activité dénitrifiante des cellules immobilisées , les caractéristiques correspondant à un fonctionnement en continu ne sont pas affectées par la nature de la source de carbone , comme le montre le tableau 7 ci-après .

Il est possible de retrouver le taux initial d'activité dénitrifiante en incubant la structure comprenant les cellules immobilisées durant 48 heures dans du milieu de MUELLER-HINTON .

La manipulation consiste à vider les compartiments des réacteurs , puis à les remplir avec de l'eau distillée stérile et à les laver durant 24 heures en faisant circuler l'eau à l'aide d'une pompe . L'eau de rinçage est ensuite remplacée par du milieu de MUELLER-HINTON et la structure contenant les cellules immobilisées est incubée in situ durant 48 heures à la température ambiante . Enfin on rince durant 48 heures avant de recommencer les expériences de dénitrification.

Sur la figure 5 , la flèche verticale indique l'étape d'activation de la structure biocatalytique.

### EXEMPLE 9 -

Diffusivités des espèces ioniques dans les structures contenant les cellules immobilisées .

a ) Détermination des coefficients de diffusion.

Les expériences de diffusion ont été effectuées à 25° C dans une cuve en Plexiglass comportant un diaphragme , similaire à celle déjà utilisée par MIGNOT et JUNTER. ( Appl.Microbiol.Biotechnol , 32 : 418-423 , 1990).

La structure membranaire séparant les deux chambres de cette cuve de diffusion est constituée d'une couche d'agar d'une épaisseur de 3 mm stérile, ou dans laquelle sont piégées des cellules de P.dénitrificans . Cette couche d'agar peut être seule ou comprise entre 2 filtres millipore ( type Durapore ) .

Les deux demi-cellules contiennent 120 $cm^3$ d'eau distillée stérile . Au début de chaque expérience , on met dans une chambre 1 $cm^3$ d'une solution saline afin d'obtenir une solution de $NaNO_3$ , $NaNO_2$ ou $NaCH_3CO_2$ d'une concentration de 7,5 mM.

Pour les expériences de diffusion de l'acétate au travers de la couche d'agar, la cuve de diffusion est désoxygénée par bullage d'azote afin d'éviter la croissance de bactéries aérobies dans la couche de gel .

La quantité totale de substance diffusante (Q) transférée d'une chambre à l'autre est mesurée en fonction du temps . Les coefficients de diffusion apparente des solutés ( Da ) sont déterminés grâce à l'équation suivante (Mignot et Junter précédemment cités ) :

$$Da = A^{-1} \ I \ C^{-1} \ (dQ/dt) \quad (1)$$

dans laquelle A et I sont la surface et l'épaisseur de la structure membranaire , C est la concentration en soluté dans le compartiment dans lequel a été initialement mise la substance diffusante et t est le temps . C est considérée comme constante durant les expériences de diffusion.

Les coefficients de diffusion effective des solutés ( $D_e$ ) à travers les composants de la structure isolés peuvent être calculés à partir de $D_a$ par l'équation suivante :

$$De = Da \ K_p^{-1} \quad (2)$$

dans laquelle $K_p$ est le coefficient de partage du soluté . Les coefficients de partage des ions nitrates , nitrites et acétates entre l'eau et la couche d'agar ou les membranes microporeuses ont été initialement déterminés ( Mignot et Junter préalablement cités ).

La résistance à la diffusion de la structure composée de la couche d'agar et des deux membranes microporeuses est la somme des résistances à la diffusion de la couche d'agar et des membranes microporeuses ( Dibdin , 1981 , Arch. Oral. Biol., 26 : 504-523 ) comme calculé par la formule suivante :

$$(L + 2l)/Da^{CS} = L/Da^A + 2l/Da^{MF} \quad (3)$$

dans laquelle $Da^{CS}$ , $Da^A$ et $Da^{MF}$ sont les coefficients de diffusion apparente du soluté respectivement dans la structure composite , la couche d'agar, et les membranes microporeuses , et l l'épaisseur des membranes microporeuses ( 150 µ ) .

b) Résultats expérimentaux .

Les coefficients de partage des ions nitrites, nitrates et acétates entre l'eau et les différents composants de la structure sont tous proches de I , à l'exception de l'acétate qui semble apparemment plus soluble dans les membranes microporeuses que dans l'eau .

Le tableau 8 ci-après résume les résultats obtenus.

Les diffusivités des trois ions dans la couche d'agar stérile sont donnés dans le tableau 9 ci-après, ce tableau indiquant aussi les coefficients de diffusion des ions dans la couche d'agar chargée avec différentes quantités de bactéries dénitrifiantes, avec ou sans période de pré-incubation de la couche de gel dans un milieu nutritif riche .

Quelle que soit la charge initiale en cellules de l'agar , les coefficients de diffusion des différents ions se répartissent de la même manière . Les diffusivités dans l'agar ne sont pas modifiées par la présence de $10^6$ bactéries par $cm^3$ de gel , mais diminuent de manière notable quand l'agar est chargé avec $10^9$ cellules par $cm^3$ .

La pré-incubation des couches d'agar avant les expériences de diffusion exerce un effet opposé sur les diffusivités , et ceci en fonction de la concentration cellulaire initiale du gel .

Une diminution des coefficients de diffusion est aussi observée pour des contenus en cellules inférieurs

( $10^6$ cellules par $cm^3$ de gel ) .Les diffusivités dans la couche d'agar chargée avec $10^9$ cellules par $cm^3$ sont supérieures après préincubation du gel par rapport aux couches non incubées.

Les coefficients de diffusion des trois ions dans la couche d'agar incluse entre deux membranes microporeuses ( structure composite ) sont inférieurs de manière significative à ceux de la couche de gel seule comme le montrent les résultats du tableau 10 ci-après .

Les résultats du tableau 10 montrent que l'acétate diffuse moins bien que les ions nitrate et nitrite dans les membranes microporeuses, la résistance à la diffusion calculée à partir de l'équation précédemment citée représentant respectivement 36,6%, 43,7% et 62,4% de la résistance à la diffusion de la structure composite pour les ions nitrate, nitrite et acétate .

En conclusion, ces exemples montrent que le procédé et le dispositif objets de la présente invention permettent d'obtenir une stabilité à long terme de l'activité dénitrifiante , tout en évitant toute contamination microbienne ou chimique de l'eau traitée durant les étapes de dénitrification .

Dans aucun des exemples d'application , la présence de P.denitrificans n'a pu être détectée dans les eaux nitratées traitées ou dans les solutions contenant la source de carbone.

A ces avantages concernant la qualité de l'eau traitée , s'ajoute le fait que la structure comportant deux compartiments distincts pour la source de carbone et les eaux à traiter , permet l'utilisation de sources de carbone alternatives, et en particulier d'eau ayant une demande biochimique d'oxygène (DBO) importante .

Il est à noter de plus que les performances des réacteurs précédemment décrits sont peu affectées par cette substitution.

Il est donc possible de combiner le traitement des eaux usées avec la dénitrification de l'eau destinée à être consommée .

La présence très faible d'ions nitrites dans les effluents des solutions apportant la source de carbone , n'est pas gênante , puisque ces solutions peuvent être utilisées en circuit fermé .

De plus , l'addition d'un second réacteur en série permet de faire baisser le taux de nitrite rémanent jusqu'à des niveaux autorisés (inférieurs à 0,1 ppm).

EP 0 445 043 A1

TABLEAU 1

Influence de l'épaisseur de la couche d'agar sur le taux de dénitrification . Conditions d'incubation $NaNO_3$ 2.5 mM , acide acétique 7.5 mM (C/N=3).

| Epaisseur de la couche d'agar ( mm) | | 3 | 5 | 7 |
|---|---|---|---|---|
| Taux maximum de dénitrification | mg NO $_3^{h-1}$ | 0,8 | 0,3 | O |
| | micromol NO $_3^{h-1}$ | 13,0 | 5,1 | O |

## TABLEAU 2

### Effet de différents rapports C/N sur les performances du réacteur

### ( débit : 7.5 cm$^3$ h$^{-1}$).

| Rapport C/N ( mol/mol) | | 2 | 3 | 4 | 6 |
|---|---|---|---|---|---|
| Taux de dénitrification rémanent | mg NO$^-_3$h$^{-1}$<br>micromoles NO$^-_3$h$^{-1}$ | 0,35<br><br>5,6 | 0,50<br><br>8,0 | 0,38<br><br>6,1 | 0,35<br><br>5,6 |
| Capacité du réacteur | micromol NO$^-$3/ cm$^2$/jour | 3,6 | 5,2 | 3,9 | 3,6 |
| Efficacité | % | 30 | 43 | 32 | 30 |

EP 0 445 043 A1

## TABLEAU 3

Performances du réacteur pour différents débits

| | | | | | | |
|---|---|---|---|---|---|---|
| Débit d'eau ($cm^3$ $h^{-1}$) | | 6,6 | 7,5 | 9,8 | 13,5 | 17,0 |
| Taux de dénitrification rémanant | mg $NO^{-3}$ $h^{-1}$<br>micromol $NO^{-3}$ $h^-$ | 0,40<br>6,5 | 0,50<br>8,0 | 0,45<br>7,3 | 0,43<br>7,0 | 0,60<br>9,6 |
| Capacité du réacteur | micromoles $NO^{-}_3$/jour/$cm^2$ | 3,0 | 5,2 | 4,7 | 4,5 | 6,2 |
| Efficacité | % | 39 | 43 | 30 | 20 | 23 |

EP 0 445 043 A1

## TABLEAU 4

Dénitrification en continu par deux réacteurs en série utilisant
l'acétate comme source de carbone

| | | Compartiment A solution nitratée à traiter | | | Compartiment B solution contenant l'acétate | | |
|---|---|---|---|---|---|---|---|
| | | $NO_3^-$ | $NO_2^-$ | $CH_3CO_2^-$ | $NO_3^-$ | $NO_2^-$ | $CH_3CO_2^-$ |
| 1er réacteur | entrée | 155 | 0 | 0 | 0 | 0 | 450 |
| | sortie | 64 | 25 | 0 | 0 | 13 | 307 |
| 2ème réacteur | entrée | 64 | 25 | 0 | 0 | 13 | 307 |
| | sortie | 29 | 25 | 0 | 0 | 2 | 232 |

EP 0 445 043 A1

## TABLEAU 5

Dénitrification et dénitratation lors du fonctionnement en continu des deux réacteurs en série, utilisant l'acétate comme source de carbone

| | | 1er réacteur | 2ème réacteur | ensemble des deux réacteurs |
|---|---|---|---|---|
| Taux de dénitrification ($\mu g\ h^{-1}$) | global | 530 | 450 | 980 |
| | par $cm^3$ d'agar | 59 | 51 | 55 |
| | par $cm^2$ de surface | 17,5 | 15 | 16 |
| Taux de dénitratation ($\mu g\ h^{-1}$) | global | 910 | 350 | 1260 |
| | par $cm^3$ d'agar | 101 | 39 | 70 |
| | par $cm^2$ de surface | 30 | 12 | 21 |
| Taux de consommation d'acétate ($\mu g\ h^{-1}$) | global | 1430 | 750 | 2180 |
| | par $cm^3$ d'agar | 159 | 83 | 121 |
| | par $cm^2$ de surface | 48 | 25 | 36 |
| Efficacité (%) | — | 34 | 45 | 64 |
| Rendement (%) | | 22 | — | 27 |

EP 0 445 043 A1

## TABLEAU 6

### Dénitrification en présence d'ions phosphates par un seul réacteur.

| Taux de Dénitrification ($\mu$g h$^{-1}$) | global | 750 |
|---|---|---|
| | par cm$^3$ d'agar | 83 |
| | par cm$^2$ de surface | 25 |
| Taux de Dénitratation ($\mu$g h$^{-1}$) | global | 780 |
| | par cm$^3$ d'agar | 87 |
| | par cm$^2$ de surface | 26 |
| Taux de consommation d'acétate ($\mu$g h$^{-1}$) | global | 1080 |
| | par cm$^3$ d'agar | 119 |
| | par cm$^2$ de surface | 36 |
| Efficacité (%) | | 48 |
| Rendement (%) | | 42 |

## TABLEAU 7

### Dénitrification en continu par un seul réacteur utilisant comme source de carbone des surnageants de boues

| | | |
|---|---|---|
| Taux de dénitrification ( $\mu$g h$^{-1}$ ) | global<br>par cm$^3$ d'agar<br>par cm$^2$ de surface | 460<br>51<br>15 |
| Taux de dénitratation ( $\mu$g h$^{-1}$ ) | global<br>par cm$^3$ d'agar<br>par cm$^2$ de surface | 720<br>80<br>24 |
| Efficacité (%) | | 30 |

EP 0 445 043 A1

## TABLEAU 8

### Répartition des espèces ioniques dans la couche d'agar et la membrane microporeuse

| Espèces ioniques | Couche d'agar | Membrane microporeuse |
|---|---|---|
| $NO_3^-$ | 1,07 | 0,99 |
| $NO_2^-$ | 1,03 | 1,00 |
| $CH_3CO_2^-$ | 1,05 | 1,17 |

EP 0 445 043 A1

## TABLEAU 9

### Diffusion des espèces ioniques dans le gel , en fonction de la charge en bactéries .

| Nombre initial de cellules / $cm^3$ de gel | Coefficients de diffusion[a] ( $\times 10^5$ $cm^2$ $s^{-1}$ ) | | |
|---|---|---|---|
| | $NO_3^-$ | $NO_2^-$ | $CH_3CO_2^-$ |
| O | 1,19 | 1,42 | 0,46 |
| $10^6$<br>$10^6$ (b) | 1,19<br>1,08 | 1,45<br>1,17 | 0,46<br>0,38 |
| $10^9$<br>$10^9$ (b) | 0,54<br>1,09 | 0,83<br>1,17 | 0,31<br>0,38 |

(a) Valeurs apparentes .
Les diffusivités effectives peuvent être obtenues comme décrit dans l'exemple 9.

(b) Après pré-incubation durant 48 heures à 30°C dans un milieu nutritif riche (milieu MUELLER-HINTON).

EP 0 445 043 A1

TABLEAU 10

Diffusion des espèces ioniques dans le gel inclu entre
deux membranes microporeuses.

|  | $NO_3^-$ | $NO_2^-$ | $CH_3CO_2^-$ |
|---|---|---|---|
| Eau (a) | 15,7 | – | 12,0 |
| Couche d'agar | 11,9 | 14,2 | 4,6 |
| Structure composite | 8,3 | 8,8 | 1,9 |
| Membrane microporeuse (b) | 2,1 | 1,8 | 0,3 |

(a) Valeurs aux dilutions infinies et à 25°C calculées à partir de la
relation de Nernst-Hartley en utilisant les valeurs des conductivités
équivalentes des ions $Na^+$ , $NO_3^-$ et $CH_3CO_2^-$ donné par Robinson et Stokes
(1959, Electrolyte solutions , 2nd , ed. Butterworths , London , p.463).

EP 0 445 043 A1

## Revendications

1. Procédé biologique pour la dénitrification des milieux liquides par réduction des nitrates , à l'aide d'une source de carbone , par un système biologique immobilisé, capable d'assurer la dénitrification, avec lequel on met en contact le liquide à dénitrifier ,caractérisé en ce que les nitrates et la source de carbone sont fournis sous la forme de deux flux liquides audit système biologique et en ce qu'on fixe à une valeur comprise entre deux seuils prédéterminés l'épaisseur de la couche d'immobilisation du système biologique.

2. Procédé selon la revendication 1, caractérisé en ce que le système biologique est immobilisé par piégeage dans un support situé entre les deux flux liquides.

3. Procédé selon l'une quelconque des revendications 1 et 2 , caractérisé en ce que le support est circonscrit par deux membranes au contact respectif des deux flux liquides , les pores de ces membranes ayant des diamètres permettant la libre circulation des substrats et des produits de la réaction de dénitrification tout en retenant le système biologique .

4. Procédé selon l'une quelconque des revendications 1 à 3 , caractérisé en ce que le système biologique est au moins un microorganisme capable de respirer les nitrates en anaérobiose.

5. Procédé selon la revendication 4 , caractérisé en ce que le microorganisme est un Pseudomonas et en particulier Pseudomonas denitrificans.

6. Procédé selon l'une quelconque des revendications 1 à 5 , caractérisé en ce que le support est un gel de polysaccharides et en particulier un gel d'agar .

7. Procédé selon l'une quelconque des revendications 1 à 6 , caractérisé en ce que la membrane a une taille de pores comprise entre 0,2 et 1 micron environ.

8. Procédé selon l'une quelconque des revendications 1 à 7 , caractérisé en ce que la source de carbone est du méthanol ou de l'acide acétique .

9. Procédé selon l'une quelconque des revendications 1 à 7, caractérisé en ce que la source de carbone est constituée par des effluents industriels ou des eaux usées contenant des composés organiques.

10. Procédé selon l'une quelconque des revendications 1 à 3, caractérisé en ce que le système biologique est un complexe multi-enzymatique.

11. Procédé selon l'une quelconque des revendications 1 à 10, caractérisé en ce que le rapport molaire C/N de la source de carbone et des nitrates est compris entre 2 et 6 et préférentiellement 2,5 et 3,5 et est ajusté par réglage de la quantité de l'un ou l'autre des flux.

12. Appareillage pour la mise en oeuvre du procédé selon la revendication 11,caractérisé en ce qu'il comprend deux compartiments A et B séparés par une couche de support (1) circonscrite sur chacune de ses deux faces par une membrane microporeuse (2) et au moins deux orifices d'entrées (6) et de sortie (7) respectifs des flux de liquide à dénitrifier et de source de carbone dans les compartiments .

13. Appareillage selon la revendication 12, caractérisé en ce que le support est constitué d'une couche d'un gel d'une épaisseur préférentiellement comprise entre 10 mm et en particulier entre 2 et 5 mm.

FIG.1

FIG.2

FIG. 3

# FIG. 4

$NO_3^-$ $(mg_x l^{-1})$
$NO_2^-$ $(mg_x l^{-1})$

Temps (h)

| ( • ) | nitrates en A |
| ( ▲ ) | nitrates en B |
| ( ○ ) | nitrites en A |
| ( △ ) | nitrites en B |

# FIG. 5

|  |  |
|---|---|
| (●) | nitrates en A |
| (▲) | nitrates en B |
| (○) | nitrites en A |
| (△) | nitrites en B |

Office européen
des brevets

**RAPPORT DE RECHERCHE EUROPEENNE**

Numero de la demande

EP 91 40 0579

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | EP-A-0 000 461 (PRODUITS CHIMIQUES UGINE)<br>* En entier * | 1,3-5,8,9,12 | C 02 F 3/28<br>C 02 F 3/10 |
| Y,D | GB-A-2 164 663 (KAO CORP.)<br>* Page 1, lignes 84-111 * | 1,3-5,8,9,12 | |
| A | EP-A-0 204 273 (NOELL GmbH)<br>* Abrégé; colonne 2, ligne 47 - colonne 3, ligne 4 * | 1,4,6 | |
| A,D | BIOTECHNOLOGY LETTERS, vol. 4, no. 6, 1988, pages 399-402; D. LE MONDE et al.: "Reduction of nitrate by Pseudomonas putrefaciens entrapped in composite agar layer/microporous membrane structures"<br>* Abrégé * | 1-4,6,8 | |

**DOMAINES TECHNIQUES RECHERCHES (Int. Cl.5)**

C 02 F

Le présent rapport a été établi pour toutes les revendications

| Lieu de la recherche | Date d'achèvement de la recherche | Examinateur |
|---|---|---|
| LA HAYE | 30-05-1991 | GONZALEZ ARIAS M.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)